# EUROPEAN PATENT SPECIFICATION

(11) **EP 3 305 945 B1**
(45) Date of publication and mention of the grant of the patent: **24.04.2019**
(21) Application number: 16800051.1
(22) Date of filing: 25.05.2016
(51) Int. Cl.: C23F 11/14, C07D 249/18, B21D 53/08, F25B 39/02, F28F 19/02

(54) **METHOD FOR PRODUCING EVAPORATOR FOR REFRIGERATION DEVICE**
VERFAHREN ZUR HERSTELLUNG EINES VERDAMPFERS FÜR KÜHLVORRICHTUNG
PROCÉDÉ DE FABRICATION D'UN ÉVAPORATEUR POUR DISPOSITIF DE RÉFRIGÉRATION

(30) Priority: 26.05.2015 JP 2015106795
(43) Date of publication of application: 11.04.2018
(73) Proprietor: Daikin Industries, Ltd., Osaka-shi, Osaka 530-8323 (JP)
(72) Inventor: AOKI, Hironori, Kita-ku, Osaka-shi Osaka 530-8323 (JP); DOI, Takashi, Kita-ku, Osaka-shi Osaka 530-8323 (JP); OKADA, Kunihiro, Kita-ku, Osaka-shi Osaka 530-8323 (JP); KATAYAMA, Hideo, Kita-ku, Osaka-shi Osaka 530-8323 (JP); TANAKA, Yuuji, Kita-ku, Osaka-shi Osaka 530-8323 (JP)
(74) Representative: Hoffmann Eitle
(86) International application number: PCT/JP2016/065429
(87) International publication number: WO 2016/190347

(56) References cited:
- JP-A- H02 157 572
- JP-A- 2001 107 204
- JP-A- 2006 213 859
- JP-A- 2008 093 713
- JP-A- 2014 132 097
- US-A1- 2006 169 170

## Description

### TECHNICAL FIELD

The present invention relates to a method of manufacturing an evaporator for a refrigeration apparatus.

### BACKGROUND ART

Conventionally, copper is sometimes used in a state where its surface is covered with a rust inhibitor in order to reduce its corrosion.

For example, in the examples described in. JP-A-2011-184714, an example in which a rust preventive film is provided on the surface of a copper wire rod is introduced. The rust inhibitor used for forming the rust preventive film is the one obtained by dissolving benzotriazole in water, alcohol, and other.

### SUMMARY OF THE INVENTION

### <Technical Problem>

Here, a copper piping used in a refrigeration apparatus often suffers from not only rusting on the surface, but also from ant nest-shaped corrosion that occurs on the outer surface due to dew condensation water generated during refrigeration cycles or rainwater or the like, and thereby a hole penetrating the copper piping may occur. If the hole is formed in the copper piping in this way, the refrigerant circulating inside will leak out.

For this reason, it is required to form the rust preventive film on the outer surface of the copper piping used in the refrigeration apparatus to reduce corrosion.

However, since the benzotriazole described in JP-A-2011-184714 is water-soluble, when the piping is used in an environment where dew condensation water is generated or in an environment the piping is exposed to rainwater, the benzotriazole dissolves out and the rust prevention effect may decrease. JP-A-2008 093713 discloses a method for joining pipes to fins when making heat exchangers by expanding the pipe using a surface treated tool. A lubricating oil containing a benzotriazole is used on the inner surfaces.

The present invention was made in view of the above points, and an object of the present invention is to provide a method of manufacturing an evaporator for a refrigeration apparatus capable of reducing corrosion.

### <Solution to Problem>

The method of manufacturing an evaporator for a refrigeration apparatus of the present invention (also referred to as "the present method" hereinafter) includes a first step (i), a second step (ii), and a third step (iii). More precisely, the present method is as follows.
1. A method of manufacturing an evaporator (23, 31) for a refrigeration apparatus (100), comprising the steps of
   (i) applying a coating agent containing a benzotriazole-based compound of the formula (I): 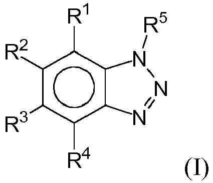 wherein R¹-R⁴ each independently are H or methyl, and R⁵ is an aliphatic C₁₋₁₈-hydrocarbon group or (CH₂)*ₙ*-N-R⁶-R⁷, *n* is an integer of 1-3, R⁶ and R⁷ each independently are an aliphatic C₁₋₁₈-hydrocarbon group,
      and a metal working oil to an outer surface of a heat transfer pipe (50) to form a rust preventive film (52);
   (ii) inserting the heat transfer pipe having the rust preventive film formed thereon into a hole (62) of a heat transfer fin (60); and
   (iii) expanding the heat transfer pipe inserted the heat transfer fin.

   Preferred embodiments of the present method are as listed below, and are further defined in the appended dependent claims and/or in the following detailed description.
2. The method as defined in item 1, wherein the heat transfer pipe is made of copper or a copper alloy, the heat transfer fin is made of aluminum or an aluminum alloy, and the metal working oil is applied to the heat transfer fins into which the heat transfer pipes are inserted in step (ii).
3. The method as defined in item 1 or 2, wherein R⁵ is CH₂-N-(CH₂-CH(C₂H₅)-CH₂-CH₂-CH₂-CH₃)₂.
4. The method as defined in item 3, wherein the benzotriazole-based compound is a mixture of N,N-bis(2-ethylhexyl)-4-methyl-1H-benzotriazole-1-methanamine and N,N-bis(2-ethylhexyl)-5-methyl-1H-benzotriazole-1-methanamine.
5. The method as defined in item 4, wherein the blending ratio of the mixture in the coating agent is 0.2-1.0 wt.-%.
6. The method as defined in item 3, wherein the benzotriazole-based compound is 1-[N,N-bis(2-ethylhexyl)aminomethyl]-1H-benzotriazole, and the blending ratio thereof in the coating agent is 0.4-1.0 wt.-%.
7. The method as defined in any of items 1-6, further comprising a step of drying the applied coating agent, wherein the drying is performed under a condition of a surface temperature of 130-200°C.
8. The method as defined in any of items 1-7, wherein the metal working oil is volatile.
9. The method as defined in any of items 1-8, further comprising a step of bending the heat transfer pipe coated with the coating agent by 180°, between step (i) and step (ii).

### <Advantageous Effects of Invention>

In the present method, even when the manufactured evaporator is exposed to dew condensation water or rainwater, since the benzotriazole-based compound is difficult to elute, the rust prevention effect can be sustained.

In the method of item 3, it becomes possible to sufficiently dissolve the benzotriazole-based compound in the metal working oil.

In the method of item 5t, the occurrence of the origin of the corrosion hole that may develop into the through hole can be effectively reduced.

In the method of item 6, the occurrence of the origin of the corrosion hole that may develop into the through hole can be effectively reduced.

In the method of item 8, it becomes possible to make the metal working oil unlikely to remain on the surface of the piping body.

### BRIEF DESCRIPTION OF THE DRAWINGS

FIG. 1 is a schematic configuration diagram of a refrigerant piping.
FIG. 2 is a schematic view of bent refrigerant pipings.
FIG. 3 is a schematic view of a heat transfer fin.
FIG. 4 is a schematic view showing a heat exchanger before U-shaped pipes are connected.
FIG. 5 is a schematic view showing a heat exchanger with U-shaped pipes connected thereto.
FIG. 6 is a schematic configuration diagram of an air conditioner.
FIG. 7 is an explanatory view showing a condition of a corrosivity check test.
FIG. 8 is a table showing test results of Examples 1a to 1e and Comparative Example 1.
FIG. 9 is a table showing test results of Examples 2a to 2e and Comparative Example 1.

### DESCRIPTION OF EMBODIMENTS

Hereinafter, an embodiment of each of a method of manufacturing a refrigerant piping, a refrigerant piping, and an air conditioner will be described as an example, but the present invention is not limited thereto.

### (1) Refrigerant piping

The refrigerant piping has a piping body and a rust preventive film.

### (2) Piping body

The piping body is a cylindrical piping, and is made of copper or a copper alloy. Examples of the copper or copper alloy include, for example, pure copper, brass, and bronze. Here, as the copper alloy, an alloy containing copper principally is preferable.

### (3) Rust preventive film

The rust preventive film is formed by applying a coating agent to an outer surface of the piping body.

Here, it is preferable that the rust preventing film is obtained by drying the coating agent applied to the outer surface of the piping body. As a method of drying, it is preferable to perform heat drying such that the surface temperature reaches 130-200°C.

The coating agent is obtained by dissolving a benzotriazole-based compound in a metal working oil. The method of dissolving the benzotriazole-based compound in the metal working oil is not particularly limited, but the benzotriazole-based compound may be dispersed by stirring with a magnetic stirrer or the like, for example.

### (3-1) Benzotriazole-based compound

The benzotriazole-based compound has a structure of the formula (I): 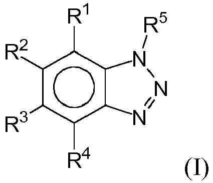 wherein R¹-R⁴ each independently are H or methyl, and R⁵ is an aliphatic C₁₋₁₈-hydrocarbon group or (CH₂)*ₙ*-N-R⁶-R⁷, *n* is an integer of 1-3, R⁶ and R⁷ each independently are an aliphatic C₁₋₁₈-hydrocarbon group. The benzotriazole-based compound may be constituted by one kind of a mixture or two or more kinds of mixtures.

In the above formula, it is preferable that R⁵ is an aliphatic C₈₋₁₈-hydrocarbon group or (CH₂)ₙ-N-R⁶-R⁷ (n is an integer of 1-3, and R⁶ and R⁷ are each independently an aliphatic C₈₋₁₈-hydrocarbon group) from the viewpoint of increasing the solubility into the metal working oil.

Here, examples of the benzotriazole-based compound include N,N-dimethylbenzotriazole-1-methanamine, N,N-diethylbenzotriazole-1-methanamine, N,N-dipropylbenzotriazole-1-methanamine, N,N-dibutylbenzotriazole-1-methanamine, N,N-dihexylbenzotriazole-1-methanamine, N,N-dioctylbenzotriazole-1-methanamine, 1-[N,N-bis(2-ethylhexyl)aminomethyl]-1H-benzotriazole, N,N-dimethyl-4-benzotriazole-1-methanamine, N,N-dimethyl-5-benzotriazole-1-methanamine, N,N-diethyl-4-benzotriazole-1-methanamine, N,N-diethyl-5-benzotriazole-1-methanamine, N,N-dipropyl-4-benzotriazole-1-methanamine, N,N-dipropyl-5-benzotriazol-1-methanamine, N,N-dibutyl-4-benzotriazol-1-methanamine, N,N-dibutyl-5-benzotriazol-1 -methanamine, N,N-dihexyl-4-benzotriazole-1 -methanamine, N,N-dihexyl-5-benzotriazole-1 -methanamine, N,N-bis(2-ethylhexyl)-4-methyl-1H-benzotriazole-1-methanamine, N,N-bis(2-ethylhexyl)-5-methyl-1H-benzotriazole-1-methanamine, N,N-dioleyl-4-methylbenzotriazole-1-methanamine, N,N-dioleyl-5-methylbenzotriazole-1-methanamine, N,N-distearyl-4-methylbenzotriazole-1-methanamine, N,N-distearyl-5-methylbenzotriazole-1-methanamine, and a mixture thereof.

Examples of preferred benzotriazole-based compounds include N,N-bis(2-ethylhexyl)-4-methyl-1H-benzotriazole-1-methanamine (CAS:80584-90-3), N,N-bis(2-ethylhexyl)-5-methyl-1H-benzotriazole-1-methanamine (CAS:80595-74-0), a mixture thereof, or 1-[N,N-bis(2-ethylhexyl)aminomethyl]-1H-benzotriazole (CAS:80301-64-0).

The benzotriazole-based compound having the above structure is composed of a BTA moiety which is relatively hydrophilic benzotriazole and a lipophilic moiety which is relatively lipophilic.

As the benzotriazole-based compound, a commercially available product may be used. Such a commercially available product is not particularly limited, but examples thereof include OA-386 (product name) manufactured by Daiwa Fine Chemicals Co., Ltd., and BT-LX (product name) manufactured by Johoku Chemical Co., Ltd.

The lower limit of the blend concentration of the benzotriazole-based compound in the coating agent is preferably ≥ 0.1 wt% from the viewpoint of making corrosion holes less likely to occur. The upper limit of the blend concentration of the benzotriazole-based compound in the coating agent is preferably ≤ 1 wt%, more preferably ≤ 0.5 wt%, because no higher limit than this seems to increase the effect of reducing the occurrence of corrosion holes, and because this limit can keep the cost low.

When a mixture of N,N-bis(2-ethylhexyl)-4-methyl-1H-benzotriazole-1-methanamine and N,N-bis(2-ethylhexyl)-5-methyl-1H-benzotriazole-1-methanamine is used as the benzotriazole-based compound, the blend concentration of the mixture in the coating agent is preferably 0.2-0.6 wt%.

Regardless of the type of the benzotriazole-based compound, from the viewpoint of reducing the occurrence of corrosion holes, the blend concentration of the benzotriazole-based compound in the coating agent is preferably 0.4-1.0 wt%, and more preferably 0.4-0.6 wt%.

### (3-2) Metal working oil

The metal working oil is not particularly limited, but it is preferably a processing oil used for metal processing and is not corrosive to (unlikely to rust) metals such as aluminum and copper.

The metal working oil preferably volatilizes at ≥ 180°C under atmospheric pressure so that it can be substantially burned off by heating and drying after processing. When the metal working oil can be substantially burned out in this way by heating and drying after processing, it is possible to reduce the generation of organic substances such as formic acid caused by deterioration or decomposition of the residual substances, leading to reduction of ant nest-shaped corrosion that is easily caused by the organic substances.

Note that when only the benzotriazole-based compound is applied to the piping body, the high viscosity tends to cause material loss during processing, and lowers the coating efficiency, and thereby the benzotriazole-based compound is coated in the state of being dissolved in the metal working oil.

The metal working oil has a kinematic viscosity of, at 40°C, preferably 1.0-5.0 mm²/s, and more preferably 1.2-2.5 mm²/s. The kinematic viscosity is a value measured in accordance with JIS K2283.

Further, the metal working oil has a density of, at 15°C, preferably 0.75-0.79 g/cm³. The density is a value measured in accordance with JIS K2249.

Also, the metal working oil preferably has an acid value of 0 mgKOH/g. Such a metal working oil preferably contains no lower carboxylic acid such as formic acid or acetic acid.

A commercially available product may be used as the above metal working oil. Examples of the commercially available product include Daphne Punch Oil AF-A series manufactured by Idemitsu Kosan Co., Ltd., and Proformer series manufactured by N·S Lubricants Co., Ltd. Among them, the product names "AF-2A", "AF-2AS" manufactured by Idemitsu Kosan Co., Ltd., and the product names "Proformer RF520", "Proformer RF510" manufactured by N·S Lubricants, Co., Ltd. are more preferable, and the product name "AF-2A" manufactured by Idemitsu Kosan Co., Ltd. is particularly preferable.

### (4) Manufacturing method of refrigerant piping and heat exchanger

The refrigerant piping is manufactured, for example, as follows.

First, the above-described piping body composed of copper or copper alloy, the above-described benzotriazole-based compound, and the metal working oil are prepared.

The prepared benzotriazole-based compound is dissolved in the metal working oil to obtain the coating agent.

This coating agent is applied to the outer surface of the piping body to form the rust preventive film.

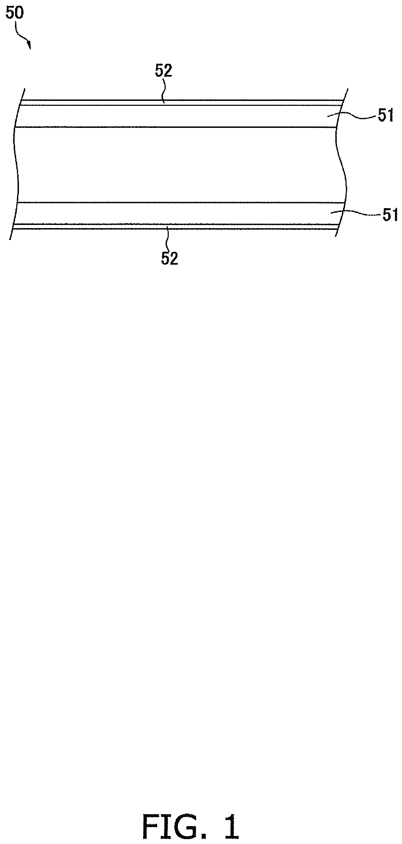

A side sectional view of the refrigerant piping 50 obtained as described above is shown in FIG. 1. Here, the refrigerant piping 50 has a cylindrical piping body 51 and a rust preventive film 52 formed on the outer surface of the piping body 51.

Note that the coating agent may be applied directly to the outer surface of the prepared piping body, but the coating application may be conducted after the outer surface of the piping body undergoes some pretreatment. Here, examples of the pretreatment of the piping body include a degreasing treatment using a solvent such as acetone and a degreasing treatment using an alkaline liquid.

Here, in order to form the rust preventive film, the coating agent applied to the outer surface of the piping body may be heated and dried. After such heating and drying, the condition is not limited, but nitrogen atoms at a position farthest from a benzene ring in a benzotriazole moiety of the benzotriazole-based compound are considered to be bound to the copper of the piping body by coordination bond.

This refrigerant piping can be used as a pipe for flowing a refrigerant inside, but the section of the refrigerant circuit where the piping is used is not particularly limited, and it may be used, for example, as a heat transfer pipe of a heat exchanger, or as a connecting pipe that connects between the main components (compressor, expansion valve, heat exchanger and the like) in the refrigerant circuit.

When the refrigerant piping is used as the heat transfer pipe in the heat exchanger of the air conditioner, the coating agent may be applied to the heat transfer fins through which the heat transfer pipe is penetrated. Hereinafter, a method of manufacturing the heat exchanger is described in a case where the refrigerant piping is used as a heat transfer pipe in a heat exchanger of an air conditioner.

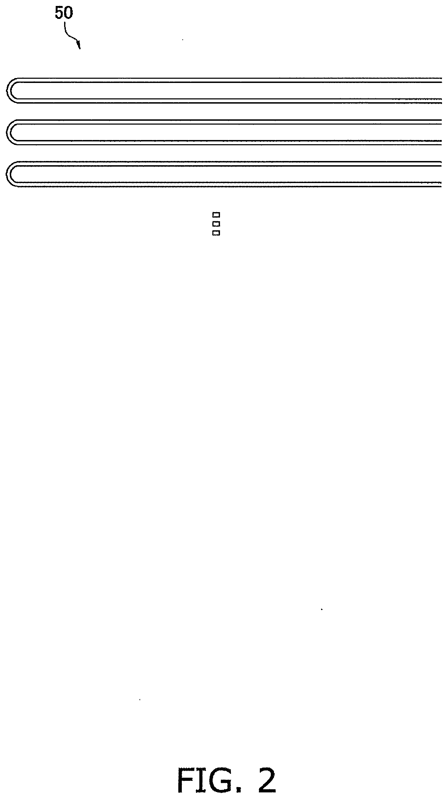

First, the coating agent obtained by dissolving the benzotriazole-based compound in the metal working oil is applied to the outer surface of the piping body 51. Then, as shown in FIG. 2, the refrigerant piping 50 coated with the coating agent (the coating agent containing the metal working oil is not yet dried) is bent by 180 degrees to form a hairpin-shaped refrigerant piping 50. Then, a plurality of such hairpin-shaped refrigerant pipings 50 are arranged side by side.

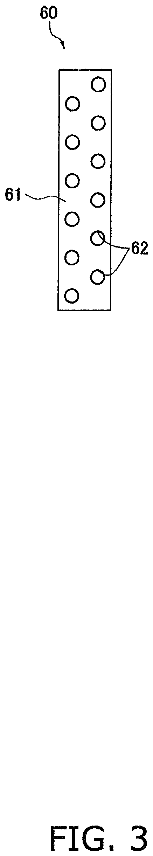

Here, as shown in FIG. 3, a plate-like heat transfer fin 60 is prepared. The heat transfer fin 60 has a fin body 61 and a plurality of holes 62 provided through the fin body 61 in the thickness direction to have the plurality of refrigerant pipings 50 penetrated therethrough. The heat transfer fin 60 is made of, for example, aluminum or an aluminum alloy. Such a heat transfer fin 60 is coated with the metal working oil. The metal working oil applied to the heat transfer fin 60 is not particularly limited, but may be the same as the metal working oil that constitutes the above-described coating agent, or may be the same as the above-described coating agent.

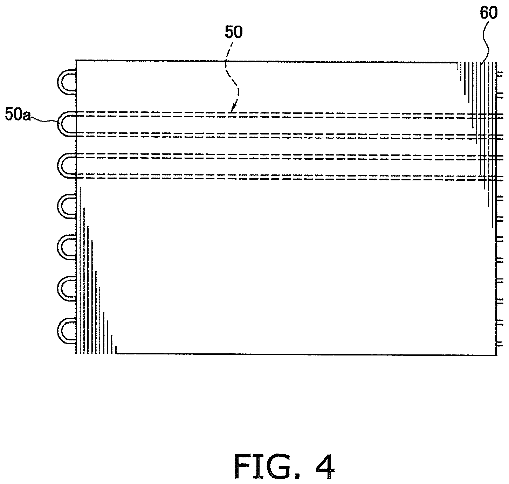

As shown in FIG. 4, the plurality of hairpin-shaped refrigerant pipings 50 arranged side by side is inserted into a plurality of the heat transfer fins 60 coated with the metal working oil. Here, after the insertion into all of the plurality of heat transfer fins 60, the refrigerant pipings 50 as the heat transfer pipes are expanded so as to increase the inner diameter of the refrigerant pipings 50 from the inside of the refrigerant pipings 50. As a result, the outer diameter of the refrigerant pipings 50 becomes equal to the inner diameter of the holes 62 of the heat transfer fins 60, and the refrigerant pipings 50 and the heat transfer fins 60 are firmly attached to each other.

The plurality of refrigerant pipings 50 and the plurality of heat transfer fins 60 obtained as described above are heated and dried. The surface temperature of the refrigerant pipings 50 and the heat transfer fins 60 at the time of heating and drying is not particularly limited, but it is preferably 130°C or more and 200°C or less, for example. Here, in the coating agent applied on the surface of the piping body 51 of the refrigerant pipings 50, the metal working oil is substantially burned off by volatilization or the like. In addition, the metal working oil and the other agent applied to the surface of the heat transfer fins 60 are also substantially burned off by volatilization or the like.

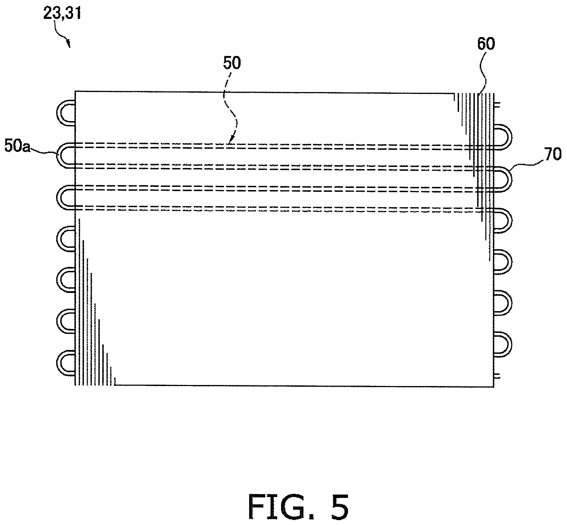

Note that, as shown in FIG. 5, a plurality of U-shaped pipes 70 are brazed and connected to a plurality of end portions of the structure taken out from a furnace, the end potions being located opposite to the bent portions 50a of the refrigerant pipings 50. The heat exchangers (23 and 31 to be described later) obtained in this way will constitute parts of the refrigerant circuit of the air conditioner.

### (5) Air conditioner including heat exchangers having refrigerant pipings

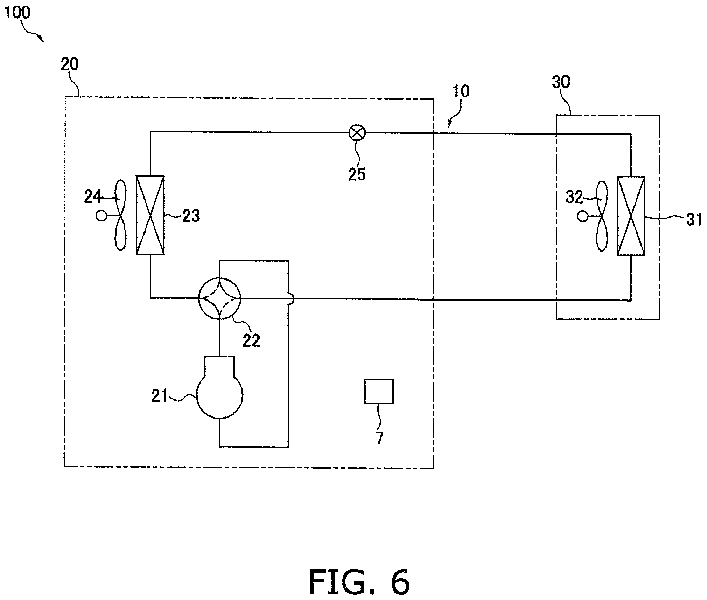

An example of an air conditioner 100 including the heat exchangers 23 and 31 having the above-described refrigerant pipings is described below with reference to FIG. 6.

The air conditioner 100 includes a refrigerant circuit 10, an outdoor fan 24, an indoor fan 32, and a control unit 7.

The refrigerant circuit 10 has a compressor 21, a four-way switching valve 22, an outdoor heat exchanger 23, an expansion valve 25, and an indoor heat exchanger 31. The refrigerant circuit 10 is configured to switch between cooling operation and heating operation by switching the connection state of the four-way switching valve 22.

Note that the indoor heat exchanger 31 and the indoor fan 32 are provided inside the indoor unit 30 that is to be installed in the air conditioning target space. The compressor 21, the four-way switching valve 22, the outdoor heat exchanger 23, the expansion valve 25, the outdoor fan 24, and the control unit 7 are provided inside the outdoor unit 20 that is to be installed outside an air conditioning target space.

During the cooling operation, the refrigerant discharged from the compressor 21 passes through one of the connection ports of the four-way switching valve 22 and is then sent to the outdoor heat exchanger 23 functioning as a radiator of the refrigerant. The refrigerant radiated heat in the outdoor heat exchanger 23 is decompressed when passing through the expansion valve 25 and sent to the indoor heat exchanger 31 functioning as an evaporator of the refrigerant. The refrigerant evaporated in the indoor heat exchanger 31 passes through another one of the connection ports of the four-way switching valve 22 and is sucked into the compressor 21 again.

During the heating operation, the refrigerant discharged from the compressor 21 passes through one of the connection ports of the four-way switching valve 22 and is then sent to the indoor heat exchanger 31 functioning as a radiator of the refrigerant. The refrigerant radiated heat in the indoor heat exchanger 31 is decompressed when passing through the expansion valve 25 and sent to the outdoor heat exchanger 23 functioning as an evaporator of the refrigerant. The refrigerant evaporated in the outdoor heat exchanger 23 passes through another one of the connection ports of the four-way switching valve 22 and is sucked into the compressor 21 again.

Note that the control unit 7 controls the drive frequency of the compressor 21, the valve opening degree of the expansion valve 25, the air volume of the outdoor fan 24, the air volume of the indoor fan 32, and the like, based on detection information of various sensors (not shown).

### EXAMPLES

Examples and Comparative Examples of refrigerant piping are shown below, but the present invention is not limited thereto.

### (Examples 1a to 1e, Comparative Example 1)

The coating agent was obtained by dissolving the benzotriazole-based compound of the product name OA-386 manufactured by Daiwa Fine Chemicals Co., Ltd. in the metalworking oil of the product name AF-2A manufactured by Idemitsu Kosan Co., Ltd.. The coating agent was applied to the outer surface of a grooved copper pipe manufactured by Shanghai Longyang Precise Composite Copper Pipe Co., Ltd. as a piping body, and was dried for 3 minutes in an environment of 130°C to obtain the refrigerant piping.

Here, an example in which the weight ratio of the benzotriazole-based compound in the coating agent was 0.1 wt% is referred to as Example 1a, an example in which the weight ratio was 0.2 wt% is referred to as Example 1b, an example in which the weight ratio was 0.3 wt% is referred to as Example 1c, an example in which the weight ratio was 0.4 wt% is referred as Example 1d, an example in which the weight ratio was 0.5 wt% is referred to as Example 1e, and an example not blended with the benzotriazole-based compound is referred to as Comparative Example 1.

### (Examples 2a to 2e)

The coating agent was obtained by dissolving the benzotriazole-based compound of the product name BT-LX manufactured by Johoku Chemical Industry Co., Ltd. in the metalworking oil with the product name AF-2A manufactured by Idemitsu Kosan Co., Ltd.. The coating agent was applied to the outer surface of the grooved copper pipe manufactured by Shanghai Longyang Precise Composite Copper Pipe Co., Ltd. as a piping body, and was dried for 3 minutes in the environment of 130°C to obtain the refrigerant piping.

Here, an example in which the weight ratio of the benzotriazole-based compound in the coating agent was 0.1 wt% is referred to as Example 2a, an example in which the weight ratio was 0.2 wt% is referred to as Example 2b, an example in which the weight ratio was 0.3 wt% is referred to as Example 2c, an example in which the weight ratio was 0.4 wt% is referred to as Example 2d, and an example in which the weight ratio was 0.5 wt% is referred to as Example 2e.

### (Corrosion Check Test)

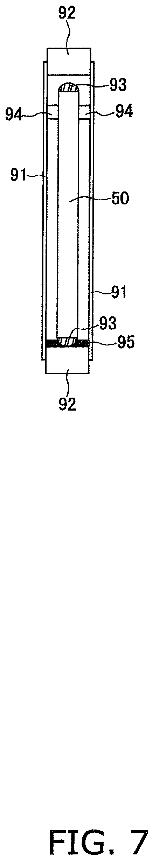

For each of these Examples 1a to 1e, 2a to 2e, and Comparative Example 1, a test was conducted to check the degree of corrosion occurrence after exposure to the environment shown in FIG.7.

As shown in FIG. 7, the refrigerant piping 50 which is the sample of each of Examples 1a to 1e, 2a to 2e, and Comparative Example 1 was placed in a cylindrical glass tube 91, and the top and bottom sides of the glass tube 91 were hermetically sealed with a silicon stopper 92. Both ends in the longitudinal direction of the refrigerant piping 50 were hermetically sealed by a hot melt resin 93 so as not to cause corrosion inside the pipe. Further, a seal tape 94 made of PTFE was stretched around the upper side of the refrigerant piping 50 and fixed the refrigerant piping 50 to the glass tube 91. Water 95 was placed in the sealed space (inside of the glass tube 91 and outside the refrigerant piping 50). Under the above circumstances, the refrigerant pipings 50 were exposed at a room temperature for 4 weeks.

Here, in Comparative Example 1, nine refrigerant pipings 50 of the same condition were prepared and tested, and in Examples 1a to 1e and 2a to 2e, eight refrigerant pipings 50 of the same condition were prepared and tested. Note that it was believed that formic acid would be generated from the organic substances such as the hot melt resin 93 in the sealed spaces to cause corrosion of the refrigerant pipings 50.

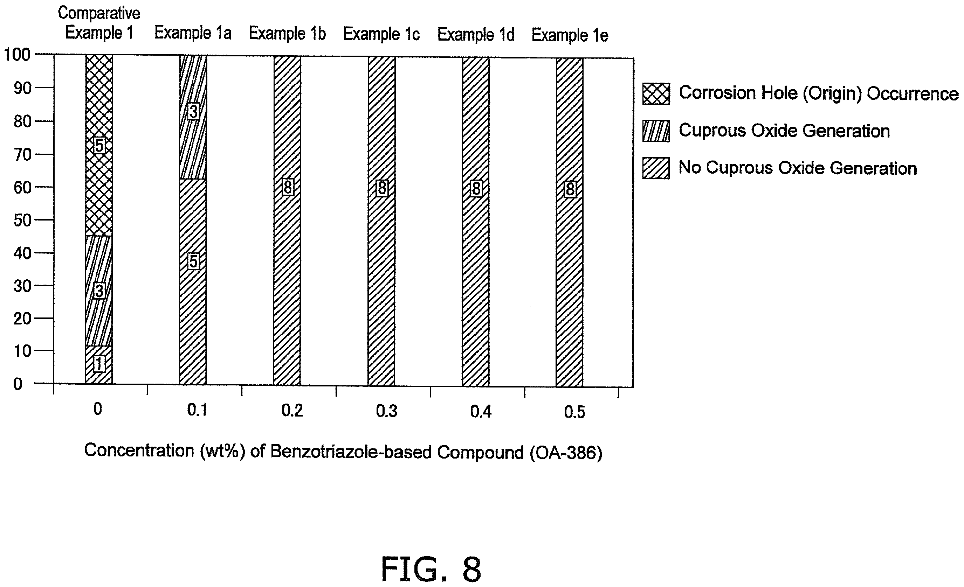

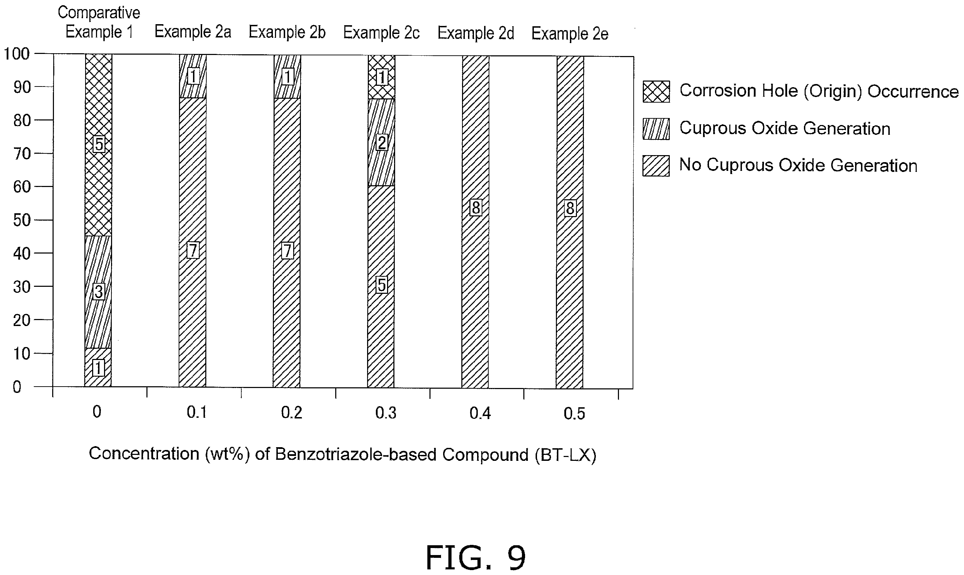

FIG. 8 and FIG. 9 show the results of each test. The numerals described in the bar-like portions in FIG. 8 and FIG. 9 each indicate the number of the refrigerant pipings 50 from which the corresponding result was obtained in the test.

Whether corrosion holes (origins) occurred or not was confirmed by visually checking for discoloration, and corrosion holes (origins) of several tens of micrometers or more were observed with a microscope. For finer corrosion holes (origins), the presence or absence of corrosion holes (origins) was confirmed by observation with a SEM. Whether cuprous oxide was generated or not was visually checked and it was determined that there was generation of cuprous oxide when dark discoloration occurred on the appearance.

As shown in FIG. 8 and FIG. 9, in any of the examples, no corrosion hole penetrating in the thickness direction of the refrigerant piping 50 occurred under the test conditions.

According to Examples 1a to 1e using the benzotriazole-based compound with the product name OA-386 manufactured by Daiwa Fine Chemicals Co., Ltd., as shown in FIG. 8, when the concentration of the benzotriazole-based compound was 0.2 wt% or more, there is no occurrence of corrosion hole (origin) or generation of cuprous oxide, indicating the concentration is preferable.

According to Examples 2a to 2e using the benzotriazole-based compound with the product name BT-LX manufactured by Johoku Chemical Co., Ltd., as shown in FIG. 9, when the concentration of the benzotriazole-based compound was 0.4 wt% or more, there is no occurrence of corrosion holes (origin) or generation of cuprous oxide, indicating the concentration is preferable.

### (6) Features

The refrigerant piping of the present embodiment is able to sustain the rust prevention effect even when exposed to dew condensation water or rainwater, since the benzotriazole-based compound is difficult to elute.

Further, when the refrigerant piping is used in the heat exchanger of the air conditioner, the refrigerant piping is able to sustain the rust prevention effect even if dew condensation water is generated on the surface of the heat exchanger when the heat exchanger is used to function as an evaporator of the refrigerant.

Furthermore, when the refrigerant piping of the present embodiment is adopted in the outdoor heat exchanger constituting the outdoor unit of the air conditioner or various pipings, the refrigerant piping is able to sustain the rust prevention effect even if it is installed outdoors and exposed to rainwater.

### REFERENCE SIGNS LIST

- 7: Control unit
- 10: Refrigerant circuit
- 21: Compressor
- 22: Four-way switching valve
- 23: Outdoor heat exchanger (heat exchanger)
- 25: Expansion valve
- 31: Indoor heat exchanger (heat exchanger)
- 50: Refrigerant piping
- 50a: Bent portion
- 51: Piping body
- 52: Rust preventive film
- 60: Heat transfer fin
- 61: Fin body
- 62: Holes
- 70: U-shaped pipe
- 100: Air conditioner

## Claims

1. A method of manufacturing an evaporator (23, 31) for a refrigeration apparatus (100) comprising the steps of
(i) applying a coating agent containing a benzotriazole-based compound of the formula (I): 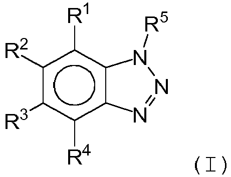
wherein R¹-R⁴ each independently are H or methyl, and R⁵ is an aliphatic C₁₋₁₈-hydrocarbon group or (CH₂)*ₙ*-N-R⁶-R⁷, *n* is an integer of 1-3, R⁶ and R⁷ each independently are an aliphatic C₁₋₁₈-hydrocarbon group,
and a metal working oil to an outer surface of a heat transfer pipe (50) to form a rust preventive film (52);
(ii) inserting the heat transfer pipe having the rust preventive film formed thereon into a hole (62) of a heat transfer fin (60); and
(iii) expanding the heat transfer pipe inserted the heat transfer fin.

2. The method of claim 1, wherein the heat transfer pipe is made of copper or a copper alloy, the heat transfer fin is made of aluminum or an aluminum alloy, and the metal working oil is applied to the heat transfer fins into which the heat transfer pipes are inserted in step (ii).

3. The method of claim 1 or 2, wherein R⁵ is CH₂-N-(CH₂-CH(C₂H₅)-CH₂-CH₂-CH₂-CH₃)₂.

4. The method of claim 3, wherein the benzotriazole-based compound is a mixture of N,N-bis(2-ethylhexyl)-4-methyl-1H-benzotriazole-1-methanamine and N,N-bis(2-ethylhexyl)-5-methyl-1H-benzotriazole-1-methanamine.

5. The method of claim 4, wherein the blending ratio of the mixture in the coating agent is 0.2-1.0 wt.-%.

6. The method of claim 3, wherein the benzotriazole-based compound is 1-[N,N-bis(2-ethylhexyl)aminomethyl]-1H-benzotriazole, and the blending ratio thereof in the coating agent is 0.4-1.0 wt.-%.

7. The method of any of claims 1-6, further comprising a step of drying the applied coating agent, wherein the drying is performed under a condition of a surface temperature of 130-200°C.

8. The method of any of claims 1-7, wherein the metal working oil is volatile.

9. The method of any of claims 1-8, further comprising a step of bending the heat transfer pipe coated with the coating agent by 180°, between step (i) and step (ii).

## Patentansprüche

1. Verfahren zur Herstellung eines Verdampfers (23, 31) für eine Kühlvorrichtung (100), umfassend die Schritte
(i) Auftragen eines Beschichtungsmittels, enthaltend eine Verbindung auf Basis von Benzotriazol der Formel (I): 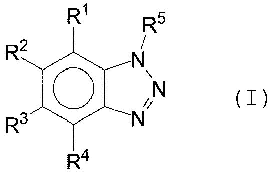
worin R¹-R⁴ jeweils unabhängig voneinander H oder Methyl sind und R⁵ eine aliphatische C₁₋₁₈-Kohlenwasserstoffgruppe oder (CH₂)*ₙ*-N-R⁶-R⁷ ist, *n* eine ganze Zahl von 1-3 ist, R⁶ und R⁷ jeweils unabhängig voneinander eine aliphatische C₁₋₁₈-Kohlenwasserstoffgruppe ist,
und ein Metallbearbeitungsöl auf eine äußere Oberfläche eines Wärmeübertragungsrohrs (50), so dass ein vor Rost schützender Film (52) gebildet wird;
(ii) Einsetzen des Wärmeübertragungsrohrs mit dem darauf gebildeten, vor Rost schützenden Film in eine Öffnung (62) einer Wärmeübertragungslamelle (60); und
(iii) Ausdehnen des in die Wärmeübertragungslamelle eingesetzten Wärmeübertragungsrohrs.

2. Verfahren gemäß Anspruch 1, worin das Wärmeübertragungsrohr aus Kupfer oder einer Kupferlegierung ist, die Wärmeübertragungslamelle aus Aluminium oder einer Aluminiumlegierung ist und das Metallbearbeitungsöl auf die Wärmeübertragungslamellen aufgetragen wird, in welche die Wärmeübertragungsrohre in Schritt (ii) eingesetzt werden.

3. Verfahren gemäß Anspruch 1 oder 2, worin R⁵ CH₂-N-(CH₂-CH(C₂H₅)-CH₂-CH₂-CH₂-CH₃)₂ ist.

4. Verfahren gemäß Anspruch 3, worin die Verbindung auf Basis von Benzotriazol ein Gemisch aus N,N-Bis(2-ethylhexyl)-4-methyl-1H-benzotriazol-1-methanamin und N,N-Bis(2-ethylhexyl)-5-methyl-1H-benzotriazol-1-methanamin ist.

5. Verfahren gemäß Anspruch 4, worin das Mischungsverhältnis des Gemischs in dem Beschichtungsmittel 0,2-1,0 Gew.-% beträgt.

6. Verfahren gemäß Anspruch 3, worin die Verbindung auf Basis von Benzotriazol 1-[N,N-Bis(2-ethylhexyl)aminomethyl]-1H-benzotriazol ist und das Mischungsverhältnis davon in dem Beschichtungsmittel 0,4-1,0 Gew.-% beträgt.

7. Verfahren gemäß mindestens einem der Ansprüche 1-6, ferner umfassend einen Schritt des Trocknens des aufgetragenen Beschichtungsmittels, worin das Trocknen unter einer Bedingung einer Oberflächentemperatur von 130-200°C durchgeführt wird.

8. Verfahren gemäß mindestens einem der Ansprüche 1-7, worin das Metallbearbeitungsöl flüchtig ist.

9. Verfahren gemäß mindestens einem der Ansprüche 1-8, ferner umfassend einen Schritt des Biegens des mit dem Beschichtungsmittel beschichteten Wärmeübertragungsrohrs um 180° zwischen Schritt (i) und (ii).

## Revendications

1. Procédé de fabrication d'un évaporateur (23, 31) pour un appareil de réfrigération (100) comprenant les étapes
(i) d'application d'un agent de revêtement contenant un composé à base de benzotriazole de la formule (I) : 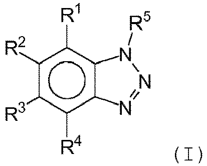
dans laquelle R¹ à R⁴ sont chacun indépendamment H ou un méthyle, et R⁵ est un groupe hydrocarbure aliphatique en C₁₋₁₈ ou (CH₂)*ₙ*-N-R⁶-R⁷, *n* est un entier de 1 à 3, R⁶ et R⁷ sont chacun indépendamment un groupe hydrocarbure aliphatique en C₁₋₁₈,
et une huile d'usinage de métal sur une surface extérieure d'un tuyau de transfert de chaleur (50) pour former un film antirouille (52) ;
(ii) d'insertion du tuyau de transfert de chaleur ayant le film antirouille formé sur celui-ci dans un trou (62) d'une ailette de transfert de chaleur (60) ; et
(iii) d'expansion du tuyau de transfert de chaleur inséré dans l'ailette de transfert de chaleur.

2. Procédé selon la revendication 1, dans lequel le tuyau de transfert de chaleur est fabriqué en cuivre ou en un alliage de cuivre, l'ailette de transfert de chaleur est fabriquée en aluminium ou en un alliage d'aluminium, et l'huile d'usinage de métal est appliquée aux ailettes de transfert de chaleur dans lesquelles les tuyaux de transfert de chaleur sont insérés dans l'étape (ii).

3. Procédé selon la revendication 1 ou 2, dans lequel R⁵ est CH₂-N-(CH₂-CH(C₂H₅)-CH₂-CH₂-CH₂-CH₃)₂.

4. Procédé selon la revendication 3, dans lequel le composé à base de benzotriazole est un mélange de N,N-bis(2-éthylhexyl)-4-méthyl-1H-benzotriazole-1-méthanamine et de N,N-bis(2-éthylhexyl)-5-méthyl-1H-benzotriazole-1-méthanamine.

5. Procédé selon la revendication 4, dans lequel le rapport de mélange du mélange dans l'agent de revêtement est de 0,2 à 1,0 % en poids.

6. Procédé selon la revendication 3, dans lequel le composé à base de benzotriazole est le 1-[N,N-bis(2-éthylhexyl)aminométhyl]-1H-benzotriazole, et le rapport de mélange de celui-ci dans l'agent de revêtement est de 0,4 à 1,0 % en poids.

7. Procédé selon l'une quelconque des revendications 1 à 6, comprenant en outre une étape de séchage de l'agent de revêtement appliqué, dans lequel le séchage est réalisé dans une condition d'une température de surface de 130 à 200 °C.

8. Procédé selon l'une quelconque des revendications 1 à 7, dans lequel l'huile d'usinage de métal est volatile.

9. Procédé selon l'une quelconque des revendications 1 à 8, comprenant en outre une étape de pliage du tuyau de transfert de chaleur revêtu avec l'agent de revêtement de 180°, entre l'étape (i) et l'étape (ii).
